Europäisches Patentamt

European Patent Office

Office européen des brevets

(11) Veröffentlichungsnummer: **0 497 849 B1**

## (12) EUROPÄISCHE PATENTSCHRIFT

(45) Veröffentlichungstag der Patentschrift: **01.03.95**

(51) Int. Cl.⁶: **C07C 303/32**, C11D 1/28

(21) Anmeldenummer: **90916269.5**

(22) Anmeldetag: **19.10.90**

(86) Internationale Anmeldenummer:
**PCT/EP90/01778**

(87) Internationale Veröffentlichungsnummer:
**WO 91/06532 (16.05.91 91/11)**

(54) **VERFAHREN ZUR SULFIERUNG UNGESÄTTIGTER FETTSÄUREGLYCERINESTER.**

(30) Priorität: **28.10.89 DE 3936001**

(43) Veröffentlichungstag der Anmeldung:
**12.08.92 Patentblatt 92/33**

(45) Bekanntmachung des Hinweises auf die
Patenterteilung:
**01.03.95 Patentblatt 95/09**

(84) Benannte Vertragsstaaten:
**DE ES FR**

(56) Entgegenhaltungen:
**DE-B- 1 246 717**
**GB-A- 793 427**

**Chemical Abstracts, Band 100, Nr. 12, 19**
**März 1984, Columbus, Ohio, US, Seite 105,**
**Zusammenfassung Nr. 87651t; PL-A-120 511**

(73) Patentinhaber: **Henkel Kommanditgesellschaft**
**auf Aktien**

**D-40191 Düsseldorf (DE)**

(72) Erfinder: **FABRY, Bernd, Dr.**
**Danziger Strasse 31**
**D-4052 Korschenbroich (DE)**
Erfinder: **SCHÄFER, Michael**
**Rathelbecker Weg 44**
**D-4006 Erkrath 1 (DE)**
Erfinder: **ANZINGER, Hermann, Dr.**
**Emil-Barth-Strasse 1**
**D-4000 Düsseldorf (DE)**

EP 0 497 849 B1

**Beschreibung**

Die Erfindung betrifft ein Verfahren zur Herstellung wäßriger Lösungen von Alkali-, Erdalkali-, Ammonium- und/oder Aminsalzen sulfierter Fettsäureglyceride durch Sulfierung ungesättigter Fettsäureglycerinester mit gasförmigem Schwefeltrioxid, Neutralisation mit Basen und anschließendem Erhitzen unter Phasentrennung sowie deren Verwendung als oberflächenaktive Mittel.

Sulfierprodukte auf Basis von ungesättigten Fettsäureglycerinestern sind seit langem bekannt. Schon 1834 wurden von Runge durch Einwirkung von Schwefelsäure auf Oliven- bzw. Ricinusöl sulfierte Öle gewonnen, die als Hilfsmittel bei der Färbung von Textilien als sogenannte "Türkischrotöle" noch heute Verwendung finden [H.Stache, H.Großmann, "Waschmittel", Springer-Verlag, Berlin-Heidelberg, 1985]. Andere sulfierte Öle finden Anwendung in der Naßveredlung von Textilien, in Handwaschpasten, als Verdickungsmittel in Lacken, als Hydrotrope für flüssige Reinigungsmittel, Korrosionsschutzmittel oder Emulgatoren für Mineralöle [Soap.Cosm.Chem.Spec. (1975) 39].

Die beiden Patentanmeldungen PL 120 511 und SU 475 391 beschreiben Verfahren zur Umsetzung von ungesättigten Triglyceriden, z.B. aus der ölsäurereichen Fraktion des Tallöls mit Schwefelsäure. Die Anlagerung von $H_2SO_4$ an die Doppelbindung ungesättigter Fettsäureglycerinester führt zur Bildung von Sulfaten, die durch -C-O-S-Bindungen charakterisiert und damit labil gegenüber dem Angriff von Säuren sind. Die Anwendung derartiger Produkte ist somit auf den neutralen bis schwach alkalischen Bereich beschränkt.

Alternativ hierzu kann die Sulfierung auch mit Oleum, d.h. mit Schwefeltrioxid durchgeführt werden, das bis zu einer Konzentration von 65 Gew.-% in Schwefelsäure gelöst vorliegt. Dieses Verfahren führt zwar neben der Bildung von Sulfaten vorwiegend zur Bildung von Sulfonaten mit säurestabiler -C-S-Bindung, ist jedoch mit dem schwerwiegenden technischen Nachteil behaftet, daß die als Lösungsmittel verwendete Schwefelsäure gemeinsam mit den erhaltenen Sulfonsäuren neutralisiert werden muß, wodurch die Produkte durch einen unerwünscht hohen Elektrolytgehalt belastet werden, welcher bei vielen Anwendungen stört.

Die deutsche Patentanmeldung DE 12 46 717 beschreibt die Sulfierung ungesättigter Fettsäureglycerinester mit gasförmigem Schwefeltrioxid im Gemisch mit einem Inertgas. Da jedoch mit steigender Reaktionstemperatur und $SO_3$-Menge neben der Sulfierung eine unerwünscht starke Verfärbung der Produkte beobachtet wird, die bei höheren Einsatzmengen des Sulfieragens bis zur Verkohlung führen kann, beschränkt sich die Durchführung des Verfahrens auf den Temperaturbereich von 0 bis 20 °C und auf Einsatzverhältnisse von Fettsäureester zu Schwefeltrioxid von 1 : 0.5 bis 1 : 1.7. Auf diese Weise sind demnach nur solche Sulfierprodukte ungesättigter Fettsäureglycerinester zugänglich, die einen geringen Gehalt an organisch gebundenem Schwefel aufweisen.

In der deutschen Patentanmeldung DE 24 37 443 A1 wird schließlich ein Verfahren zur gemeinsamen Umsetzung von ungesättigten Fettsäureglycerinestern und gesättigten Fettsäureestern mit gasförmigem Schwefeltrioxid beschrieben. Hier ist jedoch von Nachteil, daß die Herstellung homogener Mischungen beider Komponenten mit erheblichem technischen Aufwand verbunden ist und daß die Sulfierprodukte einen hohen Anteil an alpha-Fettsäuremethylestersulfonaten enthalten.

Das zu lösende technische Problem bestand nun darin, ein Verfahren zur Sulfierung von ungesättigten Fettsäureglycerinestern zu entwickeln, das nach Neutralisation zu niedrigviskosen, hellfarbigen Produkten mit hohem Anteil an organisch gebundenem Schwefel und hohem Sulfonatgehalt führt.

Gegenstand der Erfindung ist ein Verfahren zur Herstellung von wäßrigen Lösungen von Alkali-, Erdalkali-, Ammonium- und/oder Aminsalzen sulfierter Fettsäureglyceride durch Sulfierung von ungesättigten Fettsäureglycerinestern mit gasförmigem Schwefeltrioxid, nachfolgender Neutralisation mit Basen und anschließendem Erhitzen, wobei unsulfierte Anteile von der wäßrigen Tensidlösung über eine Phasentrennung abgetrennt werden können.

Unter Fettsäureglycerinestern sind die Mono-, Di- und Triester sowie deren Gemische zu verstehen, wie sie bei der Herstellung durch Veresterung von 1 Mol Glycerin mit 1 bis 3 Mol ungesättigter Fettsäure oder bei der Umesterung von ungesättigten Triglyceriden mit 0.3 bis 2 Mol Glycerin erhalten werden. Insbesondere werden diejenigen ungesättigten Fettsäureglycerinester eingesetzt, die sich aus Fettsäuren mit 16 bis 24 C-Atomen und 1 bis 5 Doppelbindungen aufbauen, so z. B. der Palmitoleinsäure, Elaidinsäure, Petroselinsäure, Chaulmograasäure, Erucasäure, Linolensäure, Arachidonsäure oder Clupanodonsäure, insbesondere aber der Ölsäure und Linolsäure, die bevorzugt mehr als 50 Gew.-% der Fettsäurekomponente ausmachen.

Die Fettsäureglycerinester können synthetischer oder natürlicher Herkunft sein. Vorzugsweise werden solche Ester verwendet, die auf Basis von Korianderöl, Chaulmograaöl, Sonnenblumenöl, Baumwollsaatöl, Olivenöl, Erdnußöl, Leinöl, Lardöl, Meadowfoamöl, Schweineschmalz oder Fischöl, insbesondere aber neuem, ölsäurereichem Rüböl gewonnen werden. Ausdrücklich können auch solche nativen Fettsäureglycerinester eingesetzt werden, deren Fettsäurekomponente sich nicht vollständig, sondern nur zum überwiegenden Teil, d. h. zu mehr als 50 Gew.-%, aus den genannten ungesättigten Fettsäuren aufbaut sowie technische Gemische verschiedener ungesättigter oder weitgehend ungesättigter Fettsäureglycerinester untereinander, sofern der Gehalt an ungesättigten Fettsäuren in den Mischungen wiederum mehr als 50 Gew.-% beträgt. Bevorzugt wird neues, ölsäurereiches Rüböl eingesetzt.

Die Sulfierung der ungesättigten Fettsäureglycerinester mit gasförmigem Schwefeltrioxid kann in der für Fettsäureniedrigalkylester bekannten Weise [J.Falbe (ed.), "Surfactants in consumer products", Springer Verlag, Berlin-Heidelberg, 1987, S. 61] erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Das Einsatzverhältnis des gasförmigen Schwefeltrioxids beträgt 0.5 bis 5.0 Mol, vorzugsweise jedoch 1.0 bis 3.0 Mol Schwefeltrioxid pro Mol Fettsäureglycerinester. Die Sulfierreaktion wird bei Temperaturen von 40 bis 98°C, insbesondere jedoch 50 bis 90°C durchgeführt.

Die bei der Sulfierung anfallenden sauren Sulfierprodukte werden in wäßrige Basen eingerührt, neutralisiert und auf einen pH-Wert von 6.5 bis 8.5 eingestellt. Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-$C_{2-4}$-Alkanolamine, beispielsweise Mono-, Di- und Triethanolamin sowie primäre, sekundäre oder tertiäre $C_{1-4}$-Alkylamine in Betracht. Die Neutralisationsbasen gelangen dabei vorzugsweise in Form 5 bis 55 gew.-%iger wäßriger Lösungen zum Einsatz, wobei 5 bis 25 gew.-%ige wäßrige Natriumhydroxidlösungen bevorzugt sind.

Die wäßrigen, die neutralisierten Sulfierprodukte enthaltenden Lösungen werden anschließend bei 80 bis 95°C, 10 bis 240 min, vorzugsweise 30 bis 120 min, auf dem Dampfbad erhitzt. Die Reaktionsmischung trennt sich dabei in eine wäßrige, die oberflächenaktive Verbindung enthaltende und eine organische, unsulfiertes Ausgangsprodukt enthaltene Phase auf, die auf einfachstem Wege, z. B. durch Dekantieren oder Separieren voneinander getrennt werden können; die organische Phase, die nur geringe Spuren Wasser enthält, kann nach Trocknung erneut in die Sulfierung eingesetzt werden. Auf diesem Wege erhält man auch bei Einsatz geinger Mengen Schwefeltrioxid Produkte mit hohem Gehalt an organisch gebundenem Schwefel. Ein zusätzlicher Vorteil besteht darin, daß sich die auf diesem Wege hergestellten Produkte durch besonders helle Farben auszeichnen.

Das Sulfierprodukt stellt ein komplexes Gemisch dar, das im wesentlichen Mono-, Di- und Triglyceridsulfonate mit innenständiger Sulfonsäuregruppierung enthält. Als Nebenprodukte bilden sich sulfonierte Fettsäuren, Glyceridsulfate, Glycerinsulfate, Glycerin und Seifen.

Die Sulfierprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfierprodukte, 0.2 bis 2 Gew.-% Wasserstoffperoxid, berechnet als 100 %ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

Die Erfindung betrifft ferner die Verwendung der durch Umsetzung von ungesättigten Fettsäureglycerinestern mit gasförmigem Schwefeltrioxid nach Neutralisation erhältlichen Produkte als oberflächenaktive Mittel.

Die folgenden Beispiele sollen den Gegenstand der Erfindung näher erläutern.

3

**Beispiele**

Tab.1: Zusammensetzung der eingesetzten Triglyceride (Edukte)
Angaben in Gew.-%

| Fettsäure | Olv | Nsb | Kor | Edn | Soj | NRb | Len | Mdw | Lrd | Fsh |
|---|---|---|---|---|---|---|---|---|---|---|
| | % | % | % | % | % | % | % | % | % | % |
| Myristinsäure | 10 | - | - | - | - | 1 | - | - | 1 | 7 |
| Palmitinsäure | 3 | 4 | 4 | 10 | 8 | - | 5 | - | 29 | 14 |
| Palmitoleinsäure | - | - | - | - | 1 | 4 | 4 | - | 3 | 11 |
| Stearinsäure | - | 4 | - | 3 | 4 | 1 | - | - | 13 | 1 |
| Ölsäure | 80 | 85 | 3 | 50 | 28 | 59 | 22 | 2 | 47 | 15 |
| Petroselinsäure | - | - | 80 | - | - | - | - | - | - | - |
| Linolsäure | 7 | 7 | 13 | 30 | 52 | 20 | 17 | 1 | 6 | 3 |
| Linolensäure | - | - | - | - | 6 | 9 | 52 | - | 1 | 1 |
| Arachinsäure | - | - | - | 3 | 1 | 1 | - | 1 | - | - |
| Gadoleinsäure | - | - | - | - | - | 2 | - | 63 | - | - |
| Behensäure | - | - | - | 2 | - | 1 | - | - | - | - |
| Erucasäure | - | - | - | - | - | 1 | - | 15 | - | - |
| Docosadiensäure | - | - | - | - | - | - | - | 18 | - | - |
| Arachidonsäure | - | - | - | - | - | - | - | - | - | 25 |
| Clupanodonsäure | - | - | - | - | - | - | - | - | - | 23 |
| Lignocerinsäure | - | - | - | 2 | - | - | - | - | - | - |
| Iodzahl* | 88 | 92 | 88 | 100 | 141 | 120 | 205 | 133 | 57 | 142 |
| Verseifungszahl | 196 | 195 | 196 | 195 | 195 | 190 | 196 | 199 | 202 | 194 |

```
Legende:  Olv  =  Olivenöl

          NSb  =  Neues Sonnenblumenöl (> 80 Gew.-% Ölsäureanteil)

          Kor  =  Korianderöl

          Edn  =  Erdnußöl

          Soj  =  Sojaöl

          NRb  =  Neues Rüböl (> 55 Gew.-% Ölsäureanteil)

          Len  =  Leinöl

          Mdw  =  Meadowfoamöl

          Lrd  =  Lardöl

          Fsh  =  Fischöl
```

Zusammensetzung gemäß GC-Analyse, Auswertung als Flächen-%. Die Iodzahl wurde nach der Kaufmann-Methode bestimmt.

Beispiele 1.1 - 1.21

**Allgemeine Arbeitsvorschrift Verfahren A : Batchsulfierung von Fettsäureglycerinestern.** In einem 1-l-Sulfierreaktor mit Mantelkühlung wurden jeweils 1 Mol des ungesättigten Fettsäureglycerinesters vorgelegt und bei 40 bis 98 °C mit 0.5 bis 5.0 Mol gasförmigem Schwefeltrioxid umgesetzt. Das Schwefeltrioxid wurde durch Erhitzen aus einer entsprechenden Menge 65 %igen Oleums ausgetrieben, auf eine Konzentration von 2 bis 5 Vol.-% verdünnt und innerhalb von 15 bis 120 min in das Ausgangsprodukt eingeleitet. Nach der Sulfierung wurde das saure Reaktionsgemisch portionsweise in wäßrige 10 gew.-%ige Base eingerührt, neutralisiert und 120 min bei 95 °C auf dem Dampfbad erhitzt, wobei sich die Lösung in eine wäßrige Tensidphase und eine organische Schicht trennte, die nicht umgesetztes Ausgangsmaterial enthielt. Nach Phasentrennung in einem Scheidetrichter wurde die organische Fraktion im Vakuum 2 h bei 80 °C getrocknet und in die Sulfierung zurückgeführt. Die wäßrige Tensidlösung wurde auf pH = 7.8 eingestellt und wies einen Feststoffgehalt von 30 % auf. Tabelle 2 gibt eine Übersicht über die eingesetzten Fettsäureglycerinester, die Reaktionsbedingungen sowie die Kenndaten der Produkte.

Beispiele 2.1. - 2.14

**Allgemeine Arbeitsvorschrift Verfahren B : Kontisulfierung von Fettsäureglycerinestern.** In einem kontinuierlich arbeitenden Fallfilmreaktor (Länge 120 cm, Querschnitt 1 cm, Eduktdurchsatz 600 g/h) mit Mantelkühlung und seitlicher $SO_3$-Begasung wurden 5.0 mol eines ungesättigten Fettsäureglycerinesters bei 40 bis 98 °C mit 5 bis 15 mol gasförmigem Schwefeltrioxid zur Reaktion gebracht. Das saure Reaktionsgemisch wurde dabei kontinuierlich in 10 gew.-%ige Base eingetragen und neutralisiert. Anschließend wurde das Produkt 120 min bei 95 °C auf dem Dampfbad erhitzt, wobei sich die Lösung in eine wäßrige Tensidphase und eine organische Schicht trennte, die nicht umgesetztes Ausgangsmaterial enthielt. Nach Phasentrennung in einem Scheidetrichter wurde die organische Fraktion im Vakuum 2 h bei 80 °C getrocknet und in die Sulfierung zurückgeführt. Die wäßrige Tensidlösung wurde auf pH = 7.8 eingestellt und wies einen Feststoffgehalt von 30 % auf. Tabelle 3 gibt eine Übersicht über die eingesetzten Fettsäureglycerinester, die Reaktionsbedingungen sowie die Kenndaten der Produkte.

Der Aniontensidgehalt (WAS) sowie die Unsulfierten Anteile (US) wurden nach den DGF-Einheitsmethoden, Stuttgart 1950-1984, H-III-10 und G-II-6b ermittelt.

Die Bestimmung der Klett-Farbzahl erfolgte nach 30 minütiger Bleiche mit 1 Gew.-% einer 35 gew.-%igen wäßrigen Wasserstoffperoxidlösung. Die Messung wurde bei einer Konzentration von 5 Gew.-% Aniontensid, pH = 7 und unter Verwendung einer 1 cm-Rundküvette sowie eines Blaufilters (400 bis 465 nm) durchgeführt.

Tab.2: Kenndaten der Beispiele nach Verfahren A

| Bsp. | Edukt | SO$_3$ Mol | Vd. % | T °C | Rkz min | Base | WAS mEq/g | US % | SO$_4{}^{2-}$ % | Farbe Klett |
|------|-------|-----|-----|----|-----|------|-----|----|------|-------|
| 1.1  | NRb | 2.0 | 2 | 80 | 48  | NaOH | 0.444 | 1.8 | 2.9 | 146 |
| 1.2  | NRb | 2.0 | 5 | 80 | 68  | NaOH | 0.444 | 2.8 | 2.7 | 158 |
| 1.3  | Olv | 2.0 | 2 | 80 | 48  | NaOH | 0.337 | 2.8 | 3.9 | 125 |
| 1.4  | Olv | 2.0 | 5 | 80 | 68  | NaOH | 0.334 | 2.8 | 3.7 | 132 |
| 1.5  | NSb | 2.0 | 2 | 80 | 48  | NaOH | 0.337 | 2.8 | 3.8 | 115 |
| 1.6  | NSb | 2.0 | 5 | 80 | 68  | NaOH | 0.338 | 2.8 | 3.2 | 119 |
| 1.7  | NRb | 0.5 | 5 | 50 | 18  | NaOH | 0.225 | 4.7 | 0.5 | 98  |
| 1.8  | NRb | 1.0 | 5 | 50 | 32  | NaOH | 0.225 | 4.1 | 1.1 | 122 |
| 1.9  | NRb | 1.5 | 5 | 50 | 51  | NaOH | 0.339 | 3.3 | 1.8 | 131 |
| 1.10 | NRb | 2.0 | 5 | 50 | 77  | NaOH | 0.339 | 3.6 | 2.4 | 149 |
| 1.11 | NRb | 0.5 | 5 | 90 | 15  | NaOH | 0.225 | 4.5 | 3.4 | 105 |
| 1.12 | NRb | 1.0 | 5 | 90 | 29  | NaOH | 0.224 | 4.1 | 0.7 | 127 |
| 1.13 | NRb | 1.5 | 5 | 90 | 45  | NaOH | 0.203 | 3.6 | 2.4 | 145 |
| 1.14 | NRb | 2.0 | 5 | 90 | 65  | NaOH | 0.334 | 3.1 | 2.7 | 160 |
| 1.15 | NRb | 2.5 | 5 | 90 | 73  | NaOH | 0.336 | 2.2 | 3.4 | 201 |
| 1.16 | NRb | 3.0 | 5 | 90 | 91  | NaOH | 0.446 | 1.9 | 3.4 | 261 |
| 1.17 | NRb | 4.0 | 5 | 90 | 108 | NaOH | 0.339 | 1.5 | 4.8 | 309 |
| 1.18 | NRb | 5.0 | 5 | 90 | 120 | NaOH | 0.446 | 0.7 | 5.1 | 396 |

| Bsp. | Edukt | $SO_3$ | Vd. | T | Rkz | Base | WAS | US | $SO_4^{2-}$ | Farbe |
|---|---|---|---|---|---|---|---|---|---|---|
| | | Mol | % | °C | min | | mEq/g | % | % | Klett |
| 1.19 | NRb | 2.0 | 5 | 90 | 65 | KOH | 0.335 | 2.9 | 2.5 | 155 |
| 1.20 | NRb | 2.0 | 5 | 90 | 65 | $NH_3$ | 0.299 | 2.8 | 2.6 | 167 |
| 1.21 | NRb | 2.0 | 5 | 90 | 65 | DEA | 0.341 | 2.9 | 2.3 | 169 |

Legende:
- $SO_3$ = Einsatzmenge Mol $SO_3$/Mol Edukt
- Vd. = $SO_3$-Konzentration im Inertgasstrom (Vol.-%)
- Rkz = Reaktionszeit
- WAS = Aniontensidgehalt
- US = Unsulfiertes (Gew.-%)
- $SO_4^{2-}$ = Sulfat, berechnet als Natriumsulfat (Gew.-%)
- DEA = Diethanolamin

Die Produkte gemäß der Beispiele 1.1 bis 1.21 wiesen einen Wassergehalt von 70 Gew.-% auf.

Tab.3

| Kenndaten der Beispiele nach Verfahren B | | | | | | | | | | |
|---|---|---|---|---|---|---|---|---|---|---|
| Bsp. | Edukt | $SO_3$ Mol | Vd. % | T °C | Rkz min | Base | WAS mEq/g | US % | $SO_4^{2-}$ % | Farbe Klett |
| 2.1 | NRb | 1.0 | 5 | 90 | - | NaOH | 0.223 | 4.6 | 3.4 | 112 |
| 2.2 | NRb | 2.0 | 5 | 90 | - | NaOH | 0.333 | 2.9 | 3.0 | 154 |
| 2.3 | NRb | 3.0 | 5 | 90 | - | NaOH | 0.445 | 1.8 | 3.3 | 244 |
| 2.4 | Olv | 2.0 | 5 | 90 | - | NaOH | 0.335 | 2.7 | 3.6 | 121 |
| 2.5 | NSb | 2.0 | 5 | 90 | - | NaOH | 0.331 | 2.8 | 3.0 | 110 |
| 2.6 | Kor | 2.0 | 5 | 90 | - | NaOH | 0.335 | 2.8 | 3.0 | 112 |
| 2.7 | Edn | 2.0 | 5 | 90 | - | NaOH | 0.337 | 2.9 | 3.4 | 136 |
| 2.8 | Soj | 2.0 | 5 | 90 | - | NaOH | 0.336 | 2.9 | 3.6 | 168 |
| 2.9 | Len | 2.0 | 5 | 90 | - | NaOH | 0.335 | 3.1 | 4.0 | 208 |
| 2.10 | Lrd | 2.0 | 5 | 90 | - | NaOH | 0.337 | 2.8 | 3.0 | 159 |
| 2.11 | Mdw | 2.0 | 5 | 90 | - | NaOH | 0.335 | 2.8 | 3.2 | 185 |
| 2.12 | Fsh | 1.0 | 5 | 90 | - | NaOH | 0.114 | 5.0 | 3.1 | 396 |
| 2.13 | Fsh[1] | 2.0 | 5 | 90 | - | NaOH | 0.329 | 4.1 | 2.9 | >800 |
| 2.14 | Fsh[2] | 2.0 | 5 | 90 | - | NaOH | 0.330 | 2.8 | 6.0 | >800 |

1) Mischung 90 Gew.-% Fischöl + 10 Gew.-% Neues Rüböl
2) Mischung 50 Gew.-% Fischöl + 50 Gew.-% Neues Rüböl

Die Produkte gemäß der Beispiele 2.1 bis 2.14 wiesen einen Wassergehalt von 70 Gew.-% auf.

## EP 0 497 849 B1

**Patentansprüche**

1. Verfahren zur Herstellung von wäßrigen Lösungen von Alkali-, Erdalkali-, Ammonium- und/oder Aminsalzen sulfierter Fettsäureglyceride, dadurch gekennzeichnet, daß man ungesättigte Fettsäureglycerinester mit gasförmigem Schwefeltrioxid umsetzt, mit Basen neutralisiert, erhitzt und die sich abscheidende nichtumgesetztes Ausgangsprodukt enthaltende organische Phase von der wäßrigen Tensidlösung abtrennt.

2. Verfahren nach Anspruch 1, dadurch gekennzeichnet, daß man von ungesättigten Fettsäure-glycerinestern synthetischer oder nativer Herkunft ausgeht, deren Anteil an ungesättigten Fettsäuren mehr als 50 Gew.-% beträgt, wobei neues, ölsäurereiches Rüböl bevorzugt ist.

3. Verfahren nach Anspruch 1 oder 2, dadurch gekennzeichnet, daß man von ungesättigten Mono-, Di- und Triglyceriden sowie deren Gemischen ausgeht, die Fettsäuren mit 16 bis 24 C-Atomen und 1 bis 5 Doppelbindungen, insbesondere solchen, deren Fettsäurekomponente zu mehr als 50 Gew.-% aus Ölsäure oder Linolsäure besteht.

4. Verfahren nach einem der Ansprüche 1 bis 3, dadurch gekennzeichnet, daß man die Sulfierung in Fallfilmreaktoren mit gasförmigem Schwefeltrioxid durchführt, das mittels eines Inertgases auf eine Konzentration von 1 bis 8, vorzugsweise 2 bis 5 Vol.-% verdünnt wird.

5. Verfahren nach einem der Ansprüche 1 bis 4, dadurch gekennzeichnet, daß das Einsatzverhältnis des Schwefeltrioxids 0.5 bis 5.0 Mol, vorzugsweise jedoch 1.0 bis 3.0 Mol Schwefeltrioxid pro Mol Fettsäureglycerinester und die Sulfiertemperatur 40 bis 98°C, vorzugsweise jedoch 50 bis 90°C beträgt.

6. Verfahren nach einem der Ansprüche 1 bis 5, dadurch gekennzeichnet, daß man die Neutralisation mit 5 bis 55 gew.-%igen wäßrigen Basen aus der von Alkalimetallhydroxiden, Erdalkalimetalloxiden und -hydroxiden, Ammoniak, Mono-, Di- und Tri-$C_{2-4}$-Alkanolaminen sowie primären, sekundären und tertiären $C_{1-4}$-Alkylaminen gebildeten Gruppe durchführt.

7. Verfahren nach einem der Ansprüche 1 bis 6, dadurch gekennzeichnet, daß man die neutralisierten Sulfierprodukte 10 bis 240 min, vorzugsweise 30 bis 120 min auf 80 bis 98°C, vorzugsweise jedoch 90 bis 95°C erhitzt, die sich bildende organisches Ausgangsmaterial enthaltende Phase von der wäßrigen Tensidlösung abtrennt und nach Trocknung in die Sulfierung zurückführt.

8. Verwendung der nach dem Verfahren nach einem der Ansprüche 1 bis 7 erhältlichen bzw. hergestellten wäßrigen Lösungen von Alkali-, Erdalkali-, Ammonium- und/oder Aminsalzen sulfierter Fettsäureglyceride als oberflächenaktive Mittel.

**Claims**

1. A process for the production of aqueous solutions of alkali metal, alkaline earth metal, ammonium and/or amine salts of sulfonated fatty acid glycerides, characterized in that unsaturated fatty acid glycerol esters are reacted with gaseous sulfur trioxide, neutralized with bases, heated and the organic phase separating which contains unreacted starting product is separated from the aqueous surfactant solution.

2. A process as claimed in claim 1, characterized in that unsaturated fatty acid glycerol esters of synthetic or natural origin in which the percentage content of unsaturated fatty acids is more than 50% by weight are used as starting material, new rapeseed oil rich in oleic acid being preferred.

3. A process as claimed in claim 1 or 2, characterized in that unsaturated mono-, di- and triglycerides and mixtures thereof containing $C_{16-24}$ fatty acids with 1 to 5 double bonds, more particularly those in which more than 50% by weight of the fatty acid component consists of oleic acid or linoleic acid, are used as starting material.

4. A process as claimed in any of claims 1 to 3, characterized in that the sulfonation is carried out in falling film reactors using gaseous sulfur trioxide diluted with an inert gas to a concentration of 1 to 8% by volume and preferably 2 to 5% by volume.

5. A process as claimed in any of claims 1 to 4, characterized in that the sulfur trioxide is used in a ratio of 0.5 to 5.0 mol and preferably 1.0 to 3.0 mol sulfur trioxide per mol fatty acid glycerol ester and the sulfonation temperature is 40 to 98°C and preferably 50 to 90°C.

6. A process as claimed in any of claims 1 to 5, characterized in that the neutralization is carried out with 5 to 55% by weight agueous bases from the group consisting of alkali metal hydroxides, alkaline earth metal oxides and hydroxides, ammonia, mono-, di- and tri-$C_{2-4}$-alkanolamines and also primary, secondary and tertiary $C_{1-4}$ alkyl amines.

7. A process as claimed in any of claims 1 to 6, characterized in that the neutralized sulfonation products are heated for 10 to 240 mins. and preferably for 30 to 120 mins. to a temperature of 80 to 98°C and preferably to a temperature of 90 to 95°C, the phase containing organic starting material is separated from the agueous surfactant solution and, after drying, is returned to the sulfonation reaction.

8. The use of the aqueous solutions of alkali metal, alkaline earth metal, ammonium and/or amine salts of sulfonated fatty acid glycerides obtainable or produced by the process claimed in any of claims 1 to 7 as surfactants.

## Revendications

1. Procédé d'obtention de solutions aqueuses de sels alcalins, de sels alcalino-terreux, de sels d'ammonium et/ou de sels d'amines de glycérides d'acides gras sulfatés, caractérisé en ce que l'on fait réagir des esters du glycérol et d'acides gras insaturés avec du trioxyde de soufre, on neutralise le produit obtenu à l'aide de bases, on le chauffe et on sépare de la solution aqueuse d'agents tensioactifs la phase organique renfermant le produit de départ n'ayant pas réagi qui précipite.

2. Procédé selon la revendication 1, caractérisé en ce que l'on part d'esters du glycérol et d'acides gras non saturés d'origine synthétique ou naturelle, dont la teneur en acides gras insaturés s'élève à plus de 50 % en poids, une huile de colza neuve riche en acide oléique étant mise en oeuvre préférentiellement.

3. Procédé selon la revendication 1 ou 2, caractérisé en ce que l'on part de mono- di- ou triglycérides insaturés ou de leurs mélanges, dérivés d'acides gras ayant de 16 à 24 atomes de carbone et de 1 à 5 doubles liaisons, en particulier de ceux dont les composants acide gras sont constitués pour plus de 50 % en poids d'acide oléique ou d'acide linoléique.

4. Procédé selon l'une des revendications 1 à 3, caractérisé en ce que l'on met en oeuvre la sulfation dans des réacteurs à pellicules tombantes en présence de trioxyde de soufre gazeux dilué au moyen d'un gaz inerte à une concentration de 1 à 8, de préférence de 2 à 5 % en volume.

5. Procédé selon l'une des revendications 1 à 4, caractérisé en ce que le trioxyde de soufre est mis en oeuvre à raison de 0,5 à 5,0 moles, de préférence cependant de 1,0 à 3,0 moles de trioxyde de soufre par mole d'ester du glycérol et d'acides gras et en ce que la température de sulfation est comprise entre 40 et 98°C, de préférence cependant entre 50 et 90°C.

6. Procédé selon l'une des revendications 1 à 5, caractérisé en ce que l'on met en oeuvre la neutralisation avec des bases aqueuses à 5 à 55 % en poids choisies dans le groupe formé par les hydroxydes de métaux alcalins, les hydroxydes et oxydes de métaux alcalino-terreux, l'ammoniac, les mono-, di- et trialcanol amines en $C_2$ à $C_4$ et les alkylamines primaires, secondaires et tertiaires en $C_1$ à $C_4$.

7. Procédé selon l'une des revendications 1 à 6, caractérisé en ce que l'on chauffe le produit de sulfation neutralisé pendant 10 à 240 minutes, de préférence 30 à 120 minutes à 80 à 98°C, de préférence cependant à 90 à 95°C, l'on sépare de la solution aqueuse d'agents tensioactifs la phase organique renfermant les produits de départ qui se forme et, après séchage, on la renvoie à l'étape de sulfation.

8. Utilisation en tant qu'agents tensioactifs des solutions aqueuses de sels alcalins, de sels alcalino-terreux, de sels d'ammonium et/ou de sels d'amines de glycérides d'acides gras sulfatés obtenues ou préparées selon le procédé conforme à l'une des revendications 1 à 7.